# EUROPEAN PATENT APPLICATION

(11) **EP 4 382 915 A1**
(43) Date of publication of application: **12.06.2024**
(21) Application number: 22212451.3
(22) Date of filing: 09.12.2022
(51) Int. Cl.: G01N 35/00, B01L 3/00, C12Q 1/6844, G01N 35/10

(54) **POINT-OF-CARE DEVICE FOR CARRYING OUT A VARIETY OF DIFFERENT BIOCHEMICAL REACTIONS**

(71) Applicant: Buzuk, Maxim, 6314 Unterägeri (CH); Buzuk, Andrey, 6314 Unterägeri (CH)
(72) Inventor: Buzuk, Maxim, 6314 Unterägeri (CH); Buzuk, Andrey, 6314 Unterägeri (CH)
(74) Representative: Schaad, Balass, Menzl & Partner AG

(57) **Abstract**

The present invention relates to a point-of-care device for carrying out a variety of different biochemical reactions including the pre-treatment of an input material comprising a sample and/or a reactant to be subjected to a biochemical reaction. The device comprises a housing (10), which encloses a housing interior (12) accessible to an operator of the device and closeable in an airtight manner, the housing interior (12) comprising at least one compartment (16a, 16b) containing at least one socket (18a, 18b) adapted to receive a cartridge (36) comprising wells (50); a dispenser (24) arranged in the compartment (16a, 16b), said dispenser being designed to transfer a liquid between wells (50) of the cartridge (36) and optionally mix liquids for providing an input material in at least some of the wells, the dispenser (24) being arranged on a motion drive (42a, 42b) for its movement in at least two coordinate axes to displace the dispenser (24) in accordance with the coordinates of the location of each well (50) of the cartridge (36); at least one thermal control unit (32) comprised in the socket (18a, 18b) and comprising energy transfer elements (52) adapted to be brought into contact with at least one well (50) of the cartridge (36) via a thermal interface of the socket (18a, 18b); and an optical system (28) arranged in the compartment (16a, 16b) for the optical determination of the content of wells (50). The device further contains a magnetic unit (68) being arranged such to lie below the cartridge (36) in the state in which the cartridge is received in the socket (18a, 18b), said magnetic unit (68) comprising at least one magnetic element (70a, 70b) and being adapted to move the at least one magnetic element (70a, 70b) in relation to at least one of the wells (50) from a distal position to a proximal position, with the magnetic element being in closer proximity to the wall of the well (50) in the proximal position than in the distal position; and a UV lamp (30) for sterilizing the compartment (16a, 16b).

## Description

The present invention relates to a point-of-care device for carrying out a variety of different biochemical reactions including the preparation of an input material comprising a sample and/or a reactant to be subjected to a biochemical reaction. The present invention further relates to a cartridge for use in the device, as well as to a point-of-care system comprising the device and the cartridge. In addition, the present invention relates to a process of carrying out a biochemical reaction using the device and the cartridge.

*In vitro* diagnostics are becoming increasingly important in healthcare. Over the recent years, the medical device industry has seen a trend towards automated testing stations of various applications and forms. Common to these devices is their purpose of supporting healthcare professional decisions while reducing the load on primary healthcare and reducing human error in performing the analysis. Due to the recent COVID-19 pandemic, PCR testing solutions rapidly gained attention as the most precise of the widely available testing approaches. During the pandemic, most healthcare systems have experienced an insufficiency in diagnostic resources and professionals.

Contrary to the testing carried out in a centralized medical laboratory, point-of-care (PoC) devices have become increasingly important as they allow samples to be analyzed in close proximity to the patient, and automated PoC devices have been instrumental in fighting the pandemic. However, they too lack capacity, and there is hence an ongoing need for a device with an elevated throughput capability.

Another limitation of the current PoC devices is their specificity to a single assay type, which is driven by the fundamental differences of typical in vitro diagnostic (IVD) procedures, including their typical read-out procedure. This limitation is aggravated by the fact that clinical guidelines often place preference on different techniques, such as ELISA, bacterial culture or colorimetric assays, for specific conditions. For example, HIV in its latent stages can be undetectable by PCR but well-seen in an anti-HIV antibody ELISA assay. Alternatively, a considerable fraction of IVD methods is based on enzyme activity assays, which are fundamentally different than molecule abundance assays. As a further indispensable laboratory method, culture assays are widely used to evaluate susceptibility of bacteria to antibiotics or of cancer cell to cytostatics.

Prior instruments for conducting thermal reactions and optical detection of reaction products typically include a metal block containing a multitude of conical reaction tubes. The metal block is heated and cooled either by a Peltier heating/cooling device or by a closed-circuit fluid heating/cooling system in which fluid flows through channels carved in the block. Such an instrument is described in US 5,333,675.

However, instruments of the type as described in US 5,333,675 have several disadvantages. Firstly, real-time thermocyclers do not allow for a suitable sample preparation or sample transfer (plate loading) to obtain an input material of high quality. This is of particular relevance for PCR, which is a sensitive technique that requires high input material quality to achieve optimal sensitivity. In practice, nucleic acid isolation or extraction required for obtaining high input material quality is either a highly manual process, which requires around an hour of highly-skilled work, or is performed by a large, separate robotic station. Such devices can therefore not be considered neither fully automatic, nor point-of-care.

Secondly, many such devices suffer from low heating and cooling rates of the thermal block, which results in very slow analyses (up to two hours) as well as sensitivity loss, since the transitional temperatures are detrimental to the amplification process, encouraging the formation of non-specific binding and primer-dimer formation.

Thirdly, the optical systems of most such devices are embodied through a mobile assembly of light diodes, photo-sensitive diodes and filters, which need to move to each reaction in the block sequentially. As a result, the optical systems can only perform light intensity analysis and not colorimetric analysis. This limits such applications as ELISA plate reading. Also, the sequential movement of the optical assembly is time-consuming.

US 6,780,617 discloses a device similar to the one available commercially under the trade name "Roche Liat Cobas". As stated in US 6,780,617, this device is designed for processing biological samples with subsequent nucleic acid amplification and detection. It includes a processing unit with at least one opening to receive a sample vessel and a plurality of processing stations positioned along the opening. The processing stations each have a compression device adapted to compress the sample vessel within the opening and thereby move the sample within the sample vessel among the processing stations. An energy transfer element can be coupled to one or more of the processing stations for transferring thermal energy to the sample at a processing station.

However, in the embodiment where the device accepts one test tube, a separate test tube for each individual patient is needed, which allows the analysis of only one patient during one run of the device. Furthermore, the device can only detect a limited number of targets, e.g. pathogen sequences (typically, up to 5 targets can be detected based on optical channel resolution and primer cross-creation) because of combined liquids used for the analysis. In another embodiment, where up to 96 samples can be analyzed in a single device, the main disadvantage is the requirement to manually move the sample vessel from the processing module (where vessels are processed in a strictly sequential order) to the thermal cycling module (where the vessels are processed in parallel), which is equivalent to the need for permanent operator presence. In addition, the device according to US 6,780,617 does not allow combining nucleic acid detection via amplification and enzyme immunoassay (ELISA) experiments in one device. A further disadvantage lies in the manufacturing of the test tubes required for the device, as these require a highly specialized sealing process.

A further device is disclosed in US 10,562,030. The key components of this device include a brushless DC motor, a door opening/closing mechanism and cartridge loading mechanism, a syringe and valve drive mechanism assembly, a sonication horn, a thermal control device and an optical detection/excitation device. Such systems can further include a communications unit configured to wirelessly communicate with a mobile device of a user to receive information about the analysis type to be performed and to relay diagnostic results.

However, the device according to 10,562,030 also has several disadvantages. Firstly, there is no direct sample introduction into the cartridge. Rather, an operator with special training is required who can introduce the sample correctly. Secondly, since combined liquids are used for the analysis (passing through one common piston pump), only one patient and a limited number of infections can be analyzed during one working cycle. Accordingly, one reaction cell restricts the number of infections tested. Further, the assay cartridge implies a complicated manufacturing process. Finally, the devise according to 10,562,030 only allows nucleic acid detection but does not support additional assays, such as ELISA or culture assays.

With regard to PCR, several designs of PoC diagnostic devices are known that employ typical, open, PCR plates or other cartridges which can collectively be defined by their conical wells and open tops. These devices are highly advantageous in their ability for parallel sample processing and analysis. Likewise, these devices can be configured to accommodate multiple molecular target detection from one sample owing to the spatially separate and disconnected wells. However, these devices have certain disadvantages:
Since the cartridges are composed of open-top wells, they need to be sealed prior to PCR initiation, as otherwise PCR reagents evaporate together with resulting amplicons, which heavily contaminate the device and even room where it is placed. The sealing step is performed by the hand of the operator, which challenges the concept of full automation.

The working chambers of these devices are not hermetic and no sterilization solution is in place, while the open cartridge allows liquid contents to aerosolise. In a context where such devices are used to diagnose infectious agents, they must be enclosed by laboratory devices that provide air filtration and other means of sterilization. This considerably limits the application of such devices in a true PoC context.

Apart from the devices discussed above, a further device is disclosed in EP 2 628 786 relating to an automated nucleic acid processor using a multi-function dispensing unit comprising;
a nozzle head provided with a suction-discharge mechanism that performs the suction and the discharge of gases, and one or two or more nozzles that detachably mount dispensing tips;
a container group having one or two or more liquid housing parts that house amplification solutions used for nucleic acid amplification and one or two or more reaction vessels;
a transfer mechanism that makes an interval between said nozzles and said container group relatively movable;
a temperature controller whereby temperature control of the interior of said reaction vessels for nucleic acid amplification is possible;
a sealing liquid and/or sealing lids housed in predetermined housing parts other than said reaction vessels of the container group that are transportable to said reaction vessels using said nozzles, which make said amplification solutions housed in the reaction vessels sealable within the reaction vessels; and
a sealing control part that controls said suction-discharge mechanism and said transfer mechanism, or said transfer mechanism, such that said sealing liquid and/or the sealing lids seal said amplification solution within the reaction vessels when the housing of said amplification solution in said reaction vessels is completed.

Like the prior art discussed further above, EP 2 628 786 does not deal with the preparation of the sample to obtain an input material of high quality. In addition to that, the processor interior is not hermetic, and no sterilization solution is in place. In a context where such devices are used to diagnose infectious agents, they must be enclosed by laboratory devices that provide air filtration. This considerably limits the application of such devices in a PoC context.

In consideration of the disadvantages of the known devices discussed above, the object of the present invention is to provide a device which allows to carry out a variety of different biochemical reactions in a safe, timesaving and fully automated manner. Specifically, the device shall allow to perform a large number of biochemical reactions simultaneously, and shall in particular allow to analyse a large number of samples by simultaneous IVD methods including the preparation of the samples for providing an input material of high quality without direct human participation.

More specifically, the present invention shall allow to perform automated, safe and highly accurate nucleic acid detection, immunoassays, colorimetric assays, culture assays and/or 3D bioprinting in one single device.

The object is solved by the device according to claim 1. Preferred embodiments of the device of the present invention are defined in the dependent claims.

According to claim 1, the present invention relates to a point-of-care device for carrying out a variety of different biochemical reactions including the pre-treatment of an input material comprising a sample and/or a reactant to be subjected to a biochemical reaction.

More specifically, the present invention relates to medicine, in particular to medicinal *in vitro* diagnostics, and more particularly concerns the design of a test system for clinical point-of-care laboratory diagnostics, including PCR, ELISA, colorimetric assays, culture assays, 3D bioprinting and isothermal nucleic acid amplification.

The term "point-of-care device" or "PoC device" as used in the context of the present invention relates to a device which allows the specific biochemical reaction for which it is designed for, in particular the medical diagnostic testing, at or near the point of care-that is, at the time and place of patient care. These devices are thus in contrast with the equipment of the former regimen in which testing was wholly or mostly confined to the medical laboratory, which entailed sending off specimens away from the point of care and then waiting hours or days to learn the results, during which time care must continue without the desired information.

As defined in claim 1, the device comprises a housing, which encloses a housing interior accessible to an operator of the device and closeable in an airtight manner, the housing interior comprising at least one compartment containing at least one socket adapted to receive a cartridge comprising wells.

The device further comprises
- a dispenser arranged in the compartment, said dispenser being designed to transfer a liquid between wells of the cartridge and optionally mix liquids for providing an input material in at least some of the wells, the dispenser being arranged on a motion drive for its movement in at least two coordinate axes to displace the dispenser in accordance with the coordinates of the location of each well of the cartridge;
- at least one thermal control unit comprised in the socket and comprising energy transfer elements adapted to be brought into contact with at least one well of the cartridge via a thermal interface of the socket; and
- an optical system arranged in the compartment for the optical determination of the content of wells.

According to the invention, the device further contains
- a magnetic unit being arranged such to lie below the cartridge in the state in which the cartridge is received in the socket, said magnetic unit comprising at least one magnetic element and being adapted to move the at least one magnetic element in relation to at least one of the wells from a distal position to a proximal position, with the magnetic element being in closer proximity to the wall of the well in the proximal position than in the distal position, and
- a UV lamp for sterilizing the compartment.

Owed to the combination of features, the device of the present invention allows the simultaneous performing of a large number of biochemical reactions in a very fast, efficient and safe manner.

As will be pointed out in further detail below, the device allows using a multi-well cartridge for samples and the possibility of pre-preparation of reagents for a multitude of reactions without direct human participation.

Specifically, the device of the present invention allows automated nucleic acid detection, immunoassays, colorimetric assays, culture assays and/or 3D bioprinting to be performed in one enclosure. Specifically, the combination of features defined in the claims allows the device to be designed in a space-saving manner.

More specifically, the device allows diagnostic processes to be performed outside of laboratory facilities (and hence in a point-of-care setting) but nonetheless in a very safe and efficient manner.

As mentioned above, the device comprises a housing, which encloses a housing interior comprising at least one compartment.

In a closed state, this compartment is hermetically sealed by a door, whereby the hermetic seal is sufficient to prevent airflow and restrict nano-scale particles in the compartment. Inside each compartment, there is at least one socket adapted to receive a cartridge comprising wells for the samples and/or reagents, typically obtained from a patient.

The term "sample" as used in the context of the present invention relates to any biological substance to be subjected to the biochemical reaction, in particular to samples to be analyzed in a diagnostic method. The term "reagent" relates to a substance or compound added to a system to cause a chemical reaction or to allow the analysis of the sample, including a solvents or buffer media. The material including the sample(s) and/or reactant(s) to be subjected to the biochemical reaction is in the context of the present invention referred to as "input material".

The dispenser arranged in the compartment is typically a robotic dispenser, which operates within each compartment, enabling transferring and mixing of the liquid content of the cartridge wells. Specifically, the dispenser is affixed to a robotic positioning system designed to position the dispenser so as to interact with each well of the cartridge. More specifically, the positioning system comprises at least one motion drive that introduces at least two degrees of motion for the dispenser unit.

According to a preferred embodiment, the dispenser comprises a nozzle, which is designed in a manner to collect plastic consumables, such as single-use plastic tips, which more specifically are stored in the cartridge. These tips prevent contamination and allow parallel processing of several samples by the device in a single analysis sequence. Through applying vertical pressure, the dispenser nozzle creates a stable, but reversible airtight lock with the consumables, in particular the single-use plastic tips.

The movement of the piston is designed to control the release frame, which enables discarding consumables back into their storing locations within the cartridge. Below a set point, a piston pulls the release frame towards the plastic consumables, ultimately pressing against them and releasing them from the dispenser nozzle. A typical liquid processing sequence may include a multitude of consumables.

According to a further preferred embodiment, the dispenser comprises at least one work volume and a dispenser piston located inside the work volume for metered extrusion of a liquid volume into a well of the cartridge, the dispenser piston being connected to a motorized drive for its motion.

Specifically, the dispenser (may contain several work volumes with respective dispenser pistons, and, accordingly, several nozzles at the end. More specifically, the dispenser can comprise a housing that hosts a number of cylindrical shaped dispenser containers, in which cavities pistons are placed, with nozzles placed at their open ends. For example, spring-loaded containers with pistons are located between two plates and providing uniform pressure and movements of the pistons. In this embodiment, the pistons can be driven by a stepper motor for synchronous dispensing. This design allows the dispenser to interact with several wells at once when used in conjunction with a cartridge with corresponding well spacing.

As will be discussed in further detail below, the thermal control unit preferably comprises a primary control unit module and a secondary control unit module. The primary thermal control unit is typically placed underneath the floor of the compartment floor. Specifically, it comprises a dedicated thermal interface as part of the socket, said interface being designed to come into tight contact with certain designated wells of the cartridge. Typically, the primary thermal control unit further comprises cooling elements which are designed to be brought into contact with the thermal interface. This ultimately allows to transmit energy to the wells or absorb energy from the wells of the cartridge.

According to a further preferred embodiment, the thermal interface is formed by the inner wall of an indentation formed in the socket and adapted to firmly receive a respective well of the cartridge, at least the lower portion of the well having a size and shape corresponding to the size and shape of the indentation, such that the inner wall of the indentation and the outer wall of the well are at least partially in direct contact with each other.

As mentioned, the thermal control unit comprising a primary control unit module and a secondary control unit module, and a secondary control unit module. The primary control unit module is destined to run a temperature program for the pre-treatment of the input material by lysis of a biological material, whereas the secondary control unit module is destined for carrying out the biochemical reaction, specifically a PCR of the prepared input material.

The optical system is typically placed above the socket and the dedicated thermal interface. Typically, it is designed such to allow measuring the intensity of coloring or luminescence in an optical channel of the system. More specifically, it comprises a combination of various wavelength light sources, excitation and emission filters and a high-sensitivity camera. The camera can for example be a high-sensitivity CCD camera, which is stationary, facing the reaction wells within the socket. Specially, the optical system can comprise an assembly containing optical cubes, which are designed to rotates around an axis perpendicularto the floor of the compartment, where each cube contains an excitation filter, a light source and an emission filter, a mirror and a motor, which controls the rotation of the assembly. The CCD matrix can for example be made of semiconductor silicone, and the filters can be made of polarized glass. According to a particularly preferred embodiment, the light source is an LED, however, other light sources can also be used, such as a laser or an incandescent lamp.

Alternative embodiments of the optical system may comprise stationary light sources surrounding the camera and moving optical filters or conversely stationary light source filter combinations and a moving camera; or a plurality of stationary fixed camera - light-source - filter combinations.

In particular in view of the use of the device for culture assays, it is particularly preferred that the optical system comprises high-resolution camera (as opposed to diode fluorescent detectors, which are common in PoC PCR devices).

The magnetic unit typically includes ate least one electromagnet or permanent magnet, and is arranged in such a way that the at least one magnetic element can interact with the contents of the cartridge, ultimately allowing to achieve an improved quality of the input material subject to the biochemical reaction, as will also be discussed in further detail below in the context of the specific working examples.

According to preferred embodiment, the magnetic unit comprises two or more magnets distanced from each other such that it allows at least one well of the cartridge to be placed between two of the magnets. However, magnetic units comprising only one magnet are also encompassed by the present invention.

The use of the magnetic unit is of particular in view of the device of the invention performing PCRs, since a broad range of input materials typically regarded as complex (due to interfering components) can be subjected to the reaction, owed to the magnetic unit enabling nucleic acid extraction and purification e.g. using silica magnetic beads.

Specifically, the magnetic unit is designed such that during analysis the magnet(s) will change position several times, either causing magnetic silica beads to attract to the walls or releasing them into solution.

In a specific instance, the following protocol can be used: upon analysis start, silica beads and, typically, a lysis buffer will be added into the sample and incubated. At this point, the magnetic silica beads will have captured the free-floating nucleic acid. The magnets come into close proximity, attract the magnetic beads and hold them against the cartridge walls while the dispenser discards unnecessary liquid. Next, the magnets are lowered, allowing the magnetic beads to be re-suspended in the washing buffer, which is a step to ensure additional cleaning from contaminants and the amplification-inhibiting lysis buffer. Again, the magnets come into close proximity to allow the dispenser to discard the washing buffer while keeping magnetics beads held against the cartridge wall. Finally, the magnets move away to allow re-suspension in the elution buffer, whereby nucleic acids are released into solution, and come into close proximity one last time to separate the (empty) silica beads from purified nucleic acids in solution.

The UV lamp is adapted to emit a radiance sufficient to sterilize typical human and animal pathogens, which further contributes to the quality of the sample, and which allows high safety standards to be met, which are of particular relevance in a point-of-care setting. In particular, the UV lamp allows the compartment to be sterilized in a very safe and fast manner before the housing is opened by the operator after the reaction(s) including the determination of the content of the wells has been carried out. Compared to gas sterilization or X-ray sterilization methods, issue which might arise when using harmful substances or radiation do not arise by using the UV lamp of the present invention.

According to a further preferred embodiment, the device further comprises a sealing unit capable of placing a sealing film over designated parts of the cartridge and applying pressure and thermal energy to the sealing film to create a hermetic seal encapsulating at least some of the wells. Specifically, the sealing unit comprises a grille, in particular an aluminum grille, thermal insulation pads and a heating element, as will be discussed in the context of the specific working examples. Specifically, the aluminum grille is in this embodiment positioned by the dispenser positioning system as well as a release bracket to a desired location in the device and above a layer of film. The grille is then pressed against the film by the dispenser positioning system and a thermal protocol is executed, whereby the film melts and forms a hermetic seal with the underlying structure, in particular the cartridge.

In a still further embodiment, the dispenser is coupled with a film-transporting mechanism, which comprises a vacuum pump, an air hose, a suction cup and a motorized drive. More specifically, the mechanism uses the motorized drive to position and press the suction cup against a sheet of film (contained in the cartridge or elsewhere), apply vacuum through the pump, pick up the film, transfer it using the dispenser positioning system and release the film at the designated location.

In particular in the context of the embodiment of the device containing a sealing unit, it can be further preferred that the dispenser comprises a conical nozzle designed such to penetrate a plastic film (i.e. a film made of a polymeric material) covering at least one of the wells, whereby during penetration an airtight, reversible seal between the nozzle and the film is formed. This plastic film can be the sealing film placed by the sealing unit disclosed above, or any other plastic film applied to the cartridge prior to its placing into the device. In order to allow for an airtight, reversible seal, the film is typically made of an elastic material.

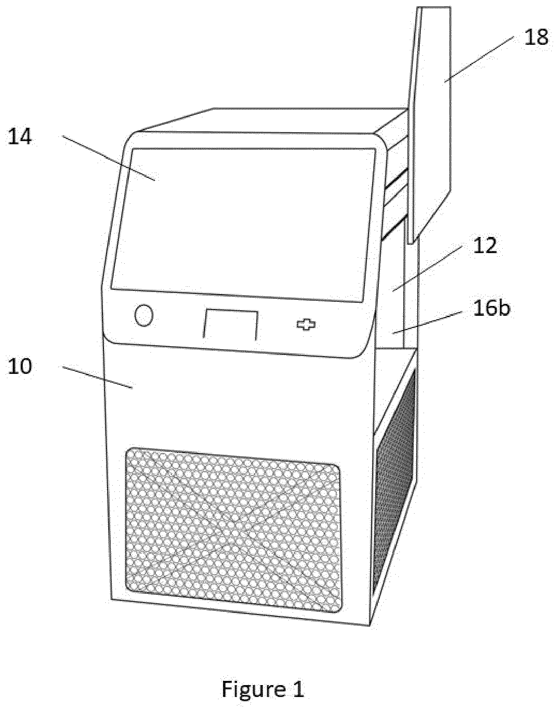

Besides the components discussed above, the device can further comprise
- a touch-sensitive display (fig. 1 (2)) arranged on the housing and acting as a user interface
- an electronic system responsible for coordinating the entire algorithm of the device
- a communication system responsible for information exchange with the device
- structural elements responsible for supporting the individual systems of the device and its appearance

According to a further aspect, the present invention also relates to a cartridge for the device discussed above. The cartridge of the present invention contains a working part, which is essentially plane and in which invaginations are formed each defining a well of the cartridge, the wells comprising an inlet opening arranged in the plane of the working part, and the shape of the inlet openings, the size of the inlet openings and/or the depth of at least some of the wells differing from each other.

Preferably, the comprises wells of the following type:
A - a well designed for the execution of the biochemical reaction (reaction well);
   and wells of at least one of the following types:
B - a well designed for containing the necessary reagent mixtures to be transferred to the well according to A (transfer well);
C - a well designed for containing consumable plastic elements (accessory transfer well);
D - a well designed for receiving analysis samples (patient sample receiving well); and
E - a well designed for receiving waste (waste well);
wherein the size and shape of the wells according to A, B, C, D and E differ from each other.

Thus, the cartridge of the invention functions as a multi-purpose working medium and a container holding a complete set of all components necessary for analysis thereby minimizing and simplifying the operator's interaction. The wells can be arranged across the working part in varying configurations depending on a certain type of analysis. In certain configurations, a single well of a certain type will be sufficient, for example, there may be only one waste well.

Further, the wells can be spatially aggregated into functional zones according to the functions of wells, for example a reaction zone, a sample preparation zone, a waste zone. Likewise, the wells can be aggregated into zones corresponding to a certain sample inserted into the cartridge. Further, the wells can be grouped in a way to expedite the algorithm (namely, the time spent by the dispenser's positioning system to execute its algorithm traveling between wells). Oppositely, there may be no traceable pattern in the placement of wells.

Preferably, the top of the cartridge (i.e. the inlet of the wells) is sealed for transportation in a filled and ready for use state, the seal being adapted such to allow the successive opening of different wells and/or different blocks of wells.

As the case may be, it can be preferable that the top of the cartridge is sealed with a silicone film, with valves being arranged in the film, said valves containing valve openings designed such that the valves are air- and liquid-tight unless the dispenser applies pressure, in which case the valve forms an airtight seal with a tip of the dispenser but allows liquid to be transferred from the dispenser into the respective well.

This embodiment allows a closed-system cartridge to be achieved, which may be beneficial in handling highly hazardous pathogens, transporting samples or discarding used cartridges. Specifically, the silicone film has substantial structural robustness to open at the valves only and also has sufficient transparency to allow optical reactions readout.

According to a further preferred embodiment, the cartridge body is equipped with an electronic identification tag, which contains information for allowing to identify at least the cartridge model (or cartridge type) and optionally also the serial number. In the context of the present invention, an electronic identification tag is understood as a means of identifying objects in which data is scanned employing optical or radio signals, such as an RFID tag (Radio Frequency Identification) tag, a barcode, and/or a QR code.

According to a further aspect, the present invention also relates to a point-of-care system for carrying out a biochemical reaction including the preparation of samples and/or reagents of the biochemical reaction comprising the device disclosed above and the cartridge disclosed above.

According to a still further aspect, the present invention also relates to a process of carrying out a biochemical reaction using the device according to any of claims 1 to 6 and the cartridge according to any of claims 7 to 11, the process comprising the step of
a. introducing the cartridge containing the samples and/or reagents into the compartment and placing it in the at least one socket;
b. transferring liquid between wells of the cartridge and optionally mix liquids such to provide a reaction input material containing a predetermined amount of a sample and/or reagent in at least some of the wells;
c. setting at least some of the wells to a specific temperature using the at least one thermal control unit
d. optionally subjecting the reaction input material to a magnetic pre-treatment using the magnetic unit;
e. determining the content of wells using the optical system; and
f. sterilizing the compartment using the UV lamp.

As mentioned, the present invention allows a variety of different biochemical reactions to be carried out, and in particular allows performing qualitative and quantitative real-time nucleic acid amplification detection, colorimetric assays and its sample preparation, ELISA assays and its sample preparation, culture assays and 3D bioprinting.

With regard to the assessment of nucleic acids using the in vitro method of polymerase chain reaction (PCR/RT-PCR) and the method of isothermal amplification Loop-mediated isothermal amplification (LAMP/RT-LAMP), the accumulated product can be detected in real time (real-time PCR) using the device of the present invention.

The device can also be used in ELISA assays, which is a method used for the qualitative or quantitative determination of proteins, in particular, viral capsids, bacterial membrane proteins, antibodies, and which is based on a specific antigen-antibody reaction where the label on the antibody serves to obtain a fluorescence or colorimetric reaction.

With regard to the fundamental steps of the ELISA protocol, the device of the invention allows *i.a.*:
- at least 3 serial washes of wells with various reagents, which are performed by the robotized dispenser;
- reagents stored in such a way that strictly prevents any mixing, which is provided by separate wells in the cartridge; and
- reading the signal, which is provided by measuring the intensity of coloring or luminescence in a certain optical channel by the optical system of the device.

The device further allows antibody-independent colorimetric assays, such as enzyme activity assays, where enzymes contained in a patient sample are exposed to an analyte and a reporter is present to indicate the rate of the reaction, allowing to draw conclusions on the enzyme activity.

With regard to the fundamental steps of the colorimetric assays, the device of the invention allows *i.a.*:
- to dilute incoming samples, which is performed by the robotic dispenser
- to store reagents in such a way that any mixing is strictly prevented, which is provided by the separate wells of the cartridge;
- to read the signal, the reading being provided by measuring the intensity of coloring or luminescence in a certain optical channel by the optical system of the device.

The device further allows to conduct culture assays which is a functional technique allowing to evaluate proliferation of microorganisms in the presence of various stimuli, such as nutrients or antibiotics. This method allows determining pathogen strain and selecting an effective antibiotic treatment.

With regard to the fundamental steps of the culture assays, the device of the invention allows *i.a.*:
- to sufficiently dilute the sample (typically, body secretes, feces or blood) to achieve the required sparsity of microorganisms; this is performed by the robotic dispenser;
- to provide a medium for the proliferation of the microorganisms, which is achieved by allocating a dedicated section of the cartridge to such medium, for example filling its wells with bacterial agar, also possibly including antibiotics in the well.
- to evenly spread the microorganisms within their medium, which is achieved by the dispenser working in couple with a plastic consumable spreader.
- to provide a suitable environment for proliferation of the microorganisms, which is maintained by the primary and secondary thermal control unit modules of the device.
- to provide image analysis to allow the readout of the test, including color and texture; this is allowed by the high-resolution camera used in the detector (as opposed to diode fluorescent detectors, which are common in PoC PCR devices).

The device can also serve as a 3D-bioprinter, which acts through sequential deposition of material, typically a cell suspension in a gel, as to achieve a certain structure, or mixture of such structures. This technique is used, for example, to generate in vitro organ models and evaluate drug toxicity; or to print cartilages with consequent implantation.

With regard to the fundamental steps of the 3D-bioprinting, the device of the invention allows *i.a.*:
- to sequentially deposit controlled volumes of material at specific spatial intervals, which is achieved by the precisely controlled robotic dispenser (for higher precision, the dispenser can work with a low-volume plastic consumable tip);
- to provide a container for housing the printing materials, which is achieved by a dedicated well of the cartridge; and
- to provide specific controlled conditions for cell survival, such as temperature and humidity, which is achieved by the interfaces of the primary and secondary thermal control unit modules of the device.

Specifically, the device is intended to be employed for in vitro diagnostics (in particular, for detection of pathogen nucleic acids in biological material, determination of hereditary diseases, genotyping, measurement of protein molecules concentration) during clinical and laboratory diagnostics and also in point-of-care applications. A nasopharyngeal or oropharyngeal swab, blood, stool can be used as the input test material. In addition to in vitro diagnostics, the device can also be used for research and laboratory purposes, for example, to determine traces of GMOs in products, genotyping laboratory animals. In the PCR context, a broad range of input sample materials typically regarded as complex (due to interfering components, for example) is allowed, in part, by the magnetic system of the device, which enables nucleic acid extraction and purification with silica magnetic beads. However, the use of this specific nucleic acid extraction principle is not mandatory. The device can accommodate alternative extraction principles, such as the direct PCR method, where samples are lysed with an amplification-compatible buffer and added to the reaction directly. Further, the device is intended for enzyme activity evaluation and for 3D-bioprinting.

### Examples

The present invention is illustrated by means of the specific working example described in the following, together with the accompanying figures of which
- Fig. 1: shows a perspective view of the device according to the present invention;
- Fig. 2: shows in a perspective view a part of the housing and of the housing interior of the device shown in Fig. 1;
- Fig. 3: shows an enlarged view of the socket and of the dispenser comprising a dispenser positioning system arranged in the housing interior shown in Fig. 2;
- Fig. 4: shows a separate view of a part of the dispenser, of which portions are illustrated transparently, thus showing a work volume of the dispenser and a dispenser piston arranged in the work volume;
- Fig. 5: shows a perspective view of the primary thermal control unit module of the thermal control unit of the device shown in Fig. 1;
- Fig. 6: shows a side view of the magnetic unit of the device shown in Fig. 1;
- Fig. 7: shows a perspective view of the optical system of the device shown in Fig. 1;
- Fig. 8: shows a perspective view of a secondary control unit module of the thermal control unit of the device shown in Fig. 1;
- Fig. 9: shows a perspective view of a cartridge of the present invention adapted for use in the device shown in Fig. 1;
- Fig. 10: shows a sectional view of a cartridge the top of which being covered with a silicone film containing valves in combination with a tip portion of the dispenser being shown in three different positions; and
- Fig. 11: shows a perspective view of part of a further embodiment of the present invention containing a sealing unit capable of placing a sealing film over designated parts of a cartridge.

According to Fig. 1, the device of the present invention comprises a housing 10, which encloses a housing interior 12 accessible to an operator of the device and closeable in an airtight manner.

In the specific embodiment shown by Fig. 1, the housing 10 roughly resembles a parallelepiped with rounded edges. Specifically, the housing comprises a load-bearing frame made of aluminum plates and extrusions. The housing and decorative elements of the panel are typically made of acrylic, plastic, and aluminum.

In the embodiment of Fig. 1, the housing comprises a display 14 for displaying information and setting the parameters of the system through a user interface. In the upper part behind the display 14, the housing interior 12 is divided into two main compartments 16a, 16b (of which the one on the right is shown as 16b is shown in Fig. 1) closed by sealed doors 18 with a magnetic lock. Electronics responsible for coordinating the elements within the system are located above and below the accessible compartments 16a, 16b.

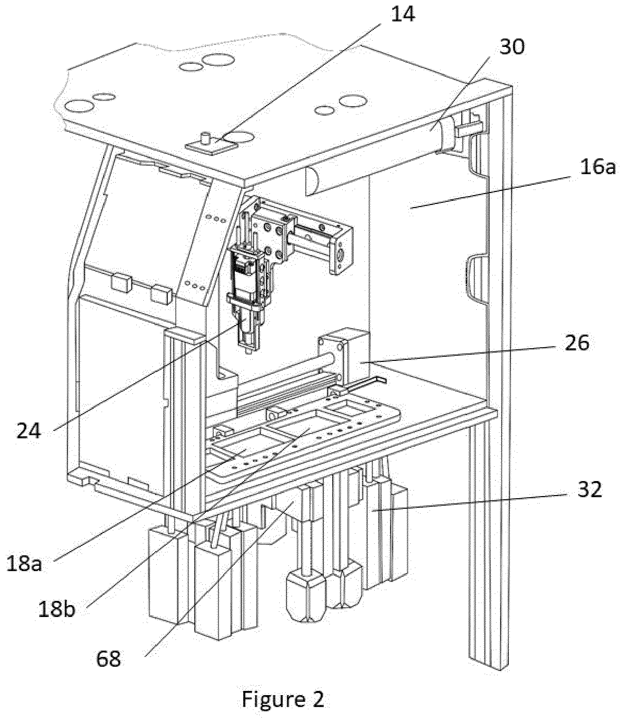

As shown in Fig. 2, each of the main compartments (of which the one on the right is shown as 16a) comprises two sockets 18a, 18b for receiving a cartridge installation. In the specific case, they are - in the case of socket 18a - positionally defined as located at the front wall (in relation to the front side 20 of the device) and - in the case of socket 18b - at the rear wall (that is, at the back side 22 of the device).

Thus, the embodiment shown allows 4 cartridges to be inserted, specifically in the left front and rear positions and in the right front and rear positions.

Each compartment comprises a dispenser 24 and a dispenser positioning system 26, which will be discussed in further detail below. In addition, each compartment further comprises an optical system 28 for the optical determination of the content of wells. (In Fig 2, only the uppermost part of the optical system is shown). In addition, each compartment is also equipped with a UV lamp 30 for disinfection of the compartment and a white light source for user convenience.

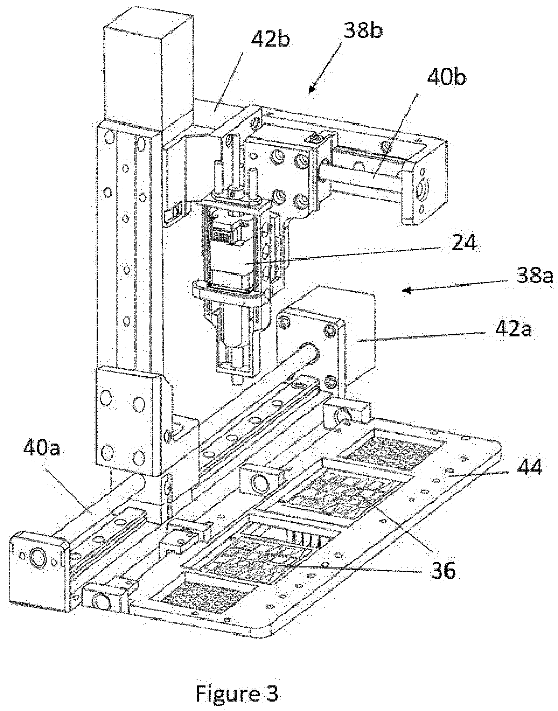

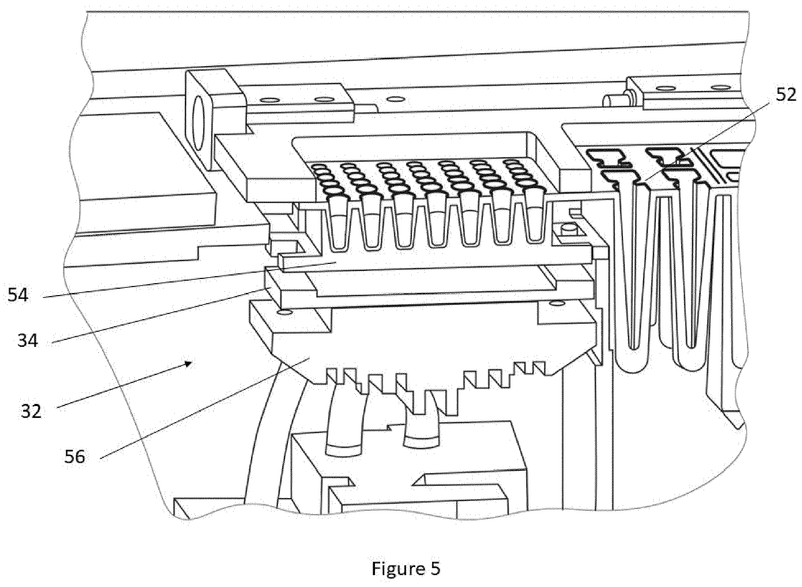

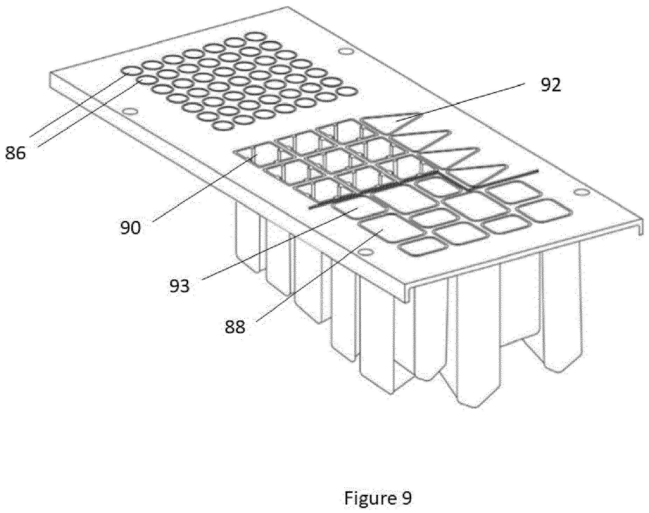

As in particular shown in Fig. 5, the sockets of each compartment further comprise at least one thermal control unit 32 comprising a primary control unit module 34 and a secondary control unit module, and a magnetic unit. The respective sockets are fully functionally independent. One cartridge 36 (of which a specific embodiment is shown in Fig. 9) can be installed in the socket in each section, as in particular shown in Fig. 3.

As mentioned, each compartment includes an independent liquid manipulation system comprising the dispenser 24 and the dispenser positioning system 26. In the embodiment shown in Fig. 2 and 3, the motion system is implemented with three orthogonal degrees of freedom (axes X, Y and Z by convention). The dispenser 24 moves in order to take specific, predetermined positions relative to the wells of each cartridge (36) and execute a sequence of manipulations.

In the embodiment shown in Fig. 2 and 3, a motion axis comprises screw-nut type linear drives 38a, 38b converting rotational displacement of stepper motors to linear displacement within the axis. The screw-nut drive is formed of a translationally fixed and rotationally free threaded screw shaft 40a, 40b, respectively, coupled with the respective motion drive 42a, 42b, and a rotationally fixed carriage with nut. The linear drives 38a, 38b are rigidly fixed to the structural frame. This combination enables precise positioning of the carriage at any point along the shaft 40a, 40b by introducing the necessary angular displacement through the stepper motor, thus enabling linear positioning. The nature of the screw-nut type transmission ensures motion system robustness, as even in the case of loss of electrical power the nut position relative to the screw is rigid. The rigidity of the fully restricted attachment mechanisms is sufficiently high, that from the perspective of the dispenser and its interaction with the cartridge it can be rigidly positioned at any necessary position.

The positioning of the cartridge inside the compartment 16 is ensured by the retention bracket 44 of the socket, and by the thermal interface of the primary thermal control module (as shown in Fig. 5). Conceptually identical linear drives Y and Z are then coupled to the carriages of axis X and axis Y correspondingly to form three degrees of translational motion.

The dispenser positioning system 26 can alternatively be based on belt-drive transmission or electromagnetic tracks. Additionally, the driving stepper motors can be replaced by continuous rotation drives, servo motors or solenoids. The different transmission and motor types can be used in conjunction to form a single motion system. Depending on the particular embodiment, a combination of differing axis types could be advantageous. For example, the base stationary axis may be chosen such as to maximize driving performance and rigidity, while for consecutive attached axes lighter weight may be a desired characteristic.

The degree of freedom choice may be dictated by the arrangement of sockets and cartridge geometry. The dispenser positioning system can conceptually be implemented with as little as two degrees of freedom or more than three.

In the embodiment shown in Fig. 2 and 3, the axes coincide with the principal axes of the device frame and enclosure, but this may not always be the case. For cases where cartridge geometry was chosen with a non-perpendicular well axis, non-orthogonal degrees of freedom may be desired. Certain embodiments may include rotation degrees of freedom. Thus, the dispenser rotation provides access to the contents of wells of complex shape, for example, when the walls of the wells are positioned at an angle to the cartridge. Thus, it is possible to provide an additional rotational axis for the dispenser parallel to the X, Y and Z axes or as a substitute to ones of the axis. Such rotation of the dispenser can be performed by a servo drive, a stepper motor with a gearbox or a stepper motor nut and levered transmission.

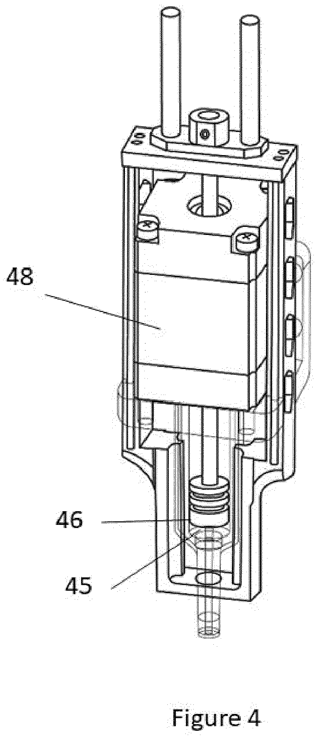

The dispenser 24 shown in Fig. 4 enables controlled and volumetrically precise reversible fluid transfer to and from the wells of the cartridge. In the specific embodiment shown in Fig. 4, the dispenser comprises a work volume 45 controlled by a piston 46, changing the lengthwise dimension of the cavity. The piston is driven by a motorized drive 48 in the form of stepper motor, rotational movement of the piston is restricted, producing direct and linear motion from the motor. The work volume changes are responsible for liquid being dispensed into or withdrawn from the cartridge wells 50 (shown in Fig. 9). A combination of individual positioning steps and liquid interactions in a sequence allows to autonomously perform all necessary sample handling operations for analysis. Thus, the dispenser 24 is capable of collecting specified exact volumes of liquid and mixing them. Functionally, this assembly is equivalent to a hand of a trained laboratory technician with a manual pi pette.

The dispenser positioning system 26 is controlled by a combination of software and hardware, which ensures precise positioning of the dispenser 24 relative to cartridge wells 50 during the interaction sequence with the liquid contents. The control system also implements motion rate control for varied motion velocity for different movements in the sequence. Likewise, this system also controls the movement and speed of the dispenser's piston 46.

As mentioned above, the socket 18a, 18b comprises a thermal control unit 32, which in the embodiment shown comprises a primary thermal control unit module 34, as shown in Fig. 5. This primary thermal control unit module 34 maintains the necessary thermal regimes for the reaction wells of the cartridges. The primary thermal control module is located below the main compartment 16a, 16b, respectively.

In the particular embodiment shown in Fig. 5, the primary thermal control unit module comprises energy transfer elements 52 in the form of a thermally conductive interface, a directionally reversible Peltier element 54 and an energy dissipation radiator 56. The thermal interface of the primary thermal control unit module 34 is placed within the socket 18a, 18b so as to provide consistent and unrestrained heat energy transfer to and from the reaction wells 50 of the cartridge 36. The geometry of the interface 52 matches the bottom surface of the reaction wells 50 in order to achieve a firm fit and facilitate energy transfer. The retention bracket 44 is responsible for vertical pressure on the cartridge 36 to ensure a tight fit. The energy dissipation radiator 56 is placed below the Peltier element 54 with directly incident work surfaces, the entire module is designed to aid bi-directional heat pumping to satisfy desired operating temperatures. The primary thermal control unit sets the desired temperature for the thermal transfer block by adjusting the Peltier current and polarity as well as ensuring an appropriate mode of operation of the energy dissipation radiator 56.

The Peltier element 54, otherwise known as a thermoelectric cooler, is an electronic device consisting of metal strips between which alternating strips of n-type and p-type semiconductors are connected. Passage of a current causes heat to be absorbed from one set of metallic strips and emitted from the other by the Peltier effect.

In essence, the primary thermal control unit module shown in Fig. 5 is a thermal heating unit with thermal control. Besides the embodiment mentioned above comprising a thermal control unit with a Peltier element and active cooler, other thermal control elements can also be used. In general, the thermal control unit includes:
- a source of heat, which, apart from the Peltier element, can be embodied by, for example, a resistive heater or a heat pump with the control side in interaction with the interface;
- a cooling system (either to dissipate heat from the hot interface itself or from the heatsink side of a heat pump), which can be presented in the form of: a passive radiator; an active heatsink; a lump heat capacity sink (such as water, dissipating heat between analysis cycles), a closed-circuit liquid cooling system with a passive or active radiator.

A primary thermal control unit module being present is of particular relevance in view of the device carrying out DNA amplification techniques and/or ELISA, both requiring that a pre-determined temperature regimen is followed.

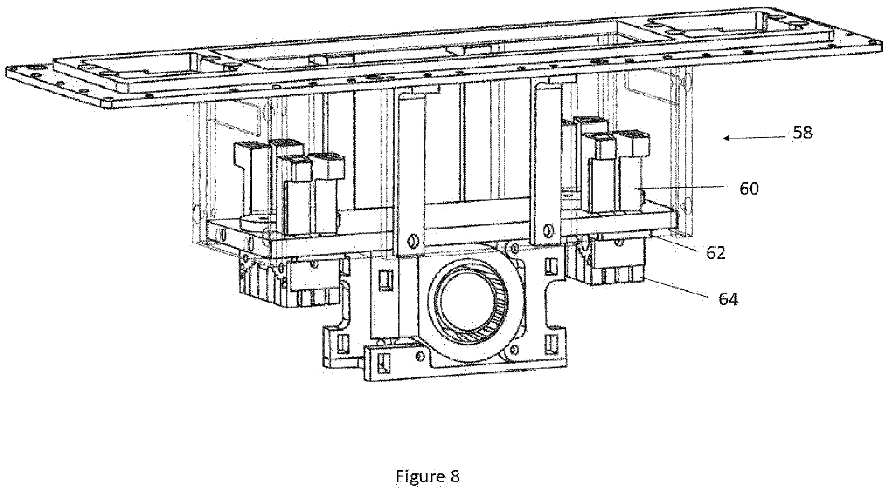

Each socket is equipped with a secondary thermal control unit module shown in Fig. 8 that forms a tight contact with a different set of wells than the primary thermal control unit module 34 described above. This secondary thermal module 58 is an independent module, supporting different operating regimes and affecting different wells of the cartridge. Similar in construction, said secondary thermal assembly contains an aluminum heat exchange interface 60 with a shape to match the curves of the wells of the cartridge that it contacts; a heating element 62 (e.g. a Peltier element or resistive heating element) that is installed with a tight contact to the heat exchange interface; a heatsink 64 and - in the embodiment shown - a fan 66. The significance of this assembly is in its operation in the nucleic acid purification process, since sample lysis and nucleic acid elution from the magnetic beads are processes that are typically more efficient when carried out at higher temperatures.

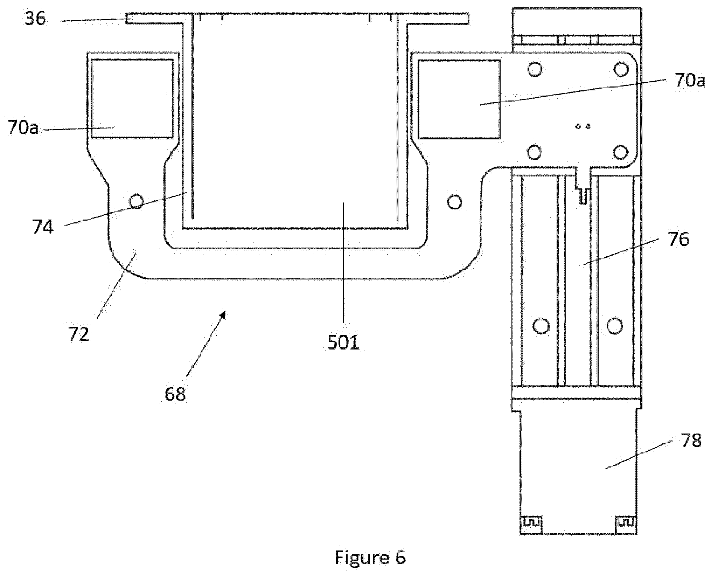

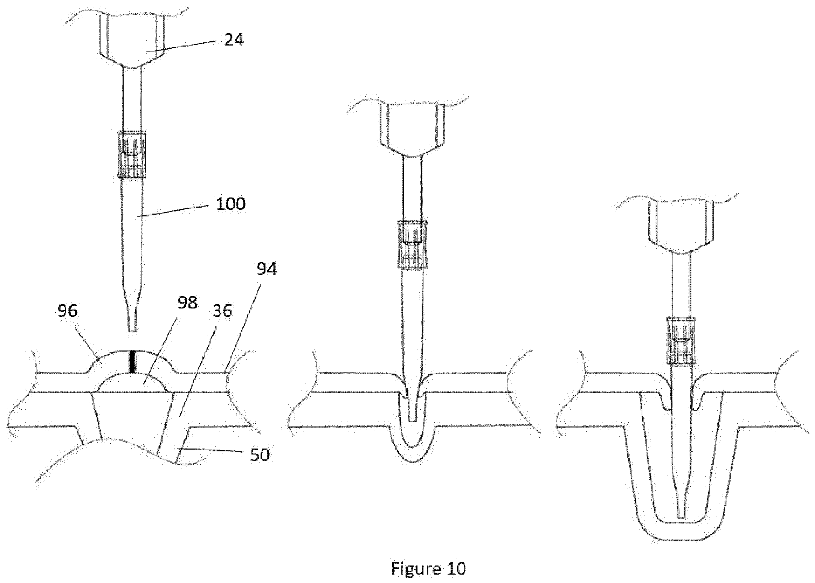

In proximity to the thermal control unit (i.e. underneath the floor or bottom of the compartment 16a, 16b, respectively) the magnetic unit 68 is arranged, as shown in Fig. 2 in combination with Fig. 6 (fig. 9 and fig. 10.) The magnetic unit supports high-quality nucleic acid extraction and purification, by influencing well contents through magnetic field effects. Said variation in the magnetic field can be achieved either by displacement of permanent magnets or electromagnets by varying their operating point, in both cases creating sufficiently strong fields within the fluid.

In the particular embodiment shown in Fig. 6, the magnetic unit is in vicinity of certain cartridge wells 501 and comprises permanent magnetic elements 70a, 70b located on a curved bracket 72, and can undergo linear movement in the vertical direction, thus allowing to move the magnetic elements from a distal position to a proximal position, in which the magnetic elements are in closer proximity to the wall 74 of the respective well 501 than in the distal position. This linear movement is controlled by a linear drive (screw-nut type) 76, analogous to a single axis of the dispenser positioning system. The stepper motor 78 is connected to the drive for moving the bracket 72 along the vertical axis along the side walls 74 of the cartridge well 501. The magnetic elements are spaced out relative to each other, cantilevered from the axis carriage, so as to surround the walls 74 of cartridge wells 501 from both sides. Once the cartridges 36 have been installed in the sockets 18a, 18b and the necessary prior sample preparation steps are completed, the bracket 72 is displaced upwards by the motion drive 76 and the magnetic elements 70a, 70b get placed at the level of the side walls 74 of the cartridge well 501. As a result, the cartridge contents experience a magnetic field far greater than when the magnetic elements 70a, 70b were lowered. In an alternative implementation, the magnetic elements 70a, 70b can be electromagnets and the magnetic field created within the cartridge can be altered by changing the power supply.

The significance of the magnetic unit is in its ability to provide a gold-standard nucleic acid extraction procedure, equivalent in nature and performance to nucleic acid extraction kits used in certified IVD kits. Biological samples often contain contaminating compounds - saliva, mucus, medicine traces. Furthermore, cellular components themselves also act as amplification inhibitors. For this reason, it is essential to follow a nucleic acid extraction protocol that provides efficient purification in order to achieve a sufficient sensitivity of the reaction. During the analysis, the magnets will change position several times, either causing the magnetic silica beads to attract to the walls or releasing them into solution.

With regard to the pre-treatment using the magnetic unit, the following protocol can be applied: upon analysis start, the lysis buffer and silica beads will be added into the sample and incubated. At this point, the magnetic silica beads will have captured the free-floating nucleic acid. The magnets (fig. 9 (3)) come into close proximity, attract the magnetic beads and hold them against the cartridge walls while the dispenser discards unnecessary liquid. Next, the magnets are lowered, allowing the magnetic beads to be re-suspended in the washing buffer, which is a step to ensure additional cleaning from contaminants and the amplification-inhibiting lysis buffer. Again, the magnets come into close proximity to allow the dispenser to discard the washing buffer while keeping magnetics beads held against the cartridge wall. Finally, the magnets move away to allow re-suspension in the elution buffer, whereby nucleic acids are released into solution, and come into close proximity one last time to separate the (empty) silica beads from purified nucleic acids in solution.

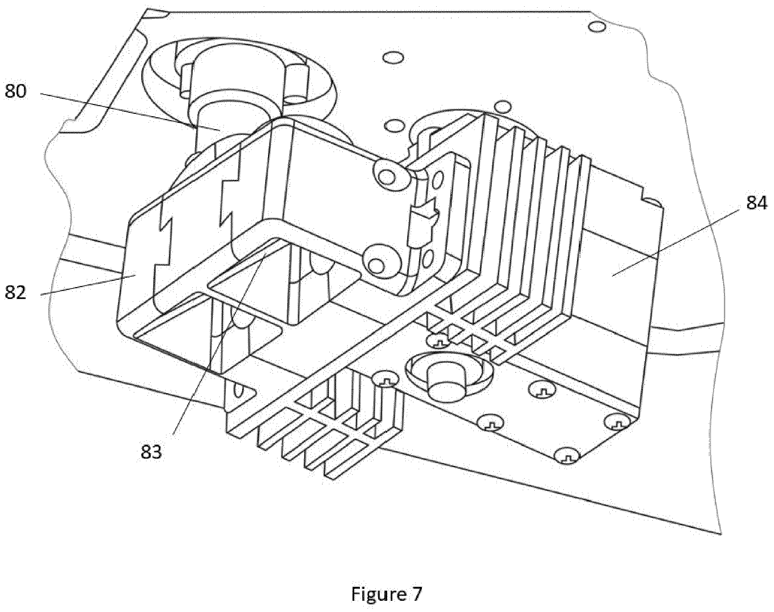

The optical system shown in Fig. 7 is responsible for measuring the optical properties of samples undergoing (usually thermal) processing in the visible and near-visible spectra. The optical system is mounted directly above the thermal interface of the primary thermal control unit module. It consists of a high-sensitivity CCD camera 80, which is stationary, facing the reaction wells within the socket; an assembly of four optical cubes 82, which rotates around an axis perpendicular to the floor of the compartment, where each cube contains an excitation filter (not shown), a light source (not shown) and an emission filter (not shown), a mirror 83, a motor 84, which controls the rotation of the optical block assembly.

A consumable plastic cartridge as shown in Fig. 9 is used with the claimed device. It simultaneously serves as a transport container for reagents, a container for accepting samples, a container for conducting reactions, a waste container, a container for disinfection, and a container for transferring additional plastic products needed during the sample preparation process.

In the specific embodiment shown in Fig. 9, the cartridge may be a container that combines reaction wells 86, transfer wells 88, waste wells 90, patient sample receiving wells 92, and accessory transfer wells 93, of additional consumables.

The wells are arranged across the working part in varying configurations depending on a certain type of analysis. In the specific embodiment shown in Fig. 9, the wells are spatially aggregated into functional zones according to the functions of wells, for example a reaction zone, a sample preparation zone, a waste zone.

In a preferable embodiment, the cartridge for the claimed device is a container made of a polymeric material that has a flat working surface in the form of a plate. Wells with inlets are made as invaginations to this plate. The shape of the wells and the geometry of their walls, as well as the location within the cartridge, are determined by their functional purpose, and the inlet openings of at least a part of the wells have different shapes in terms of the well inlets; also, different wells have different depths.

The cartridge may be equipped with an electronic identification tag, which should at least contain the cartridge model. An electronic identification label can also be placed on the cartridge packaging and scanned before unpacking.

The top of the cartridge can be closed with a foil cover for the safety of pre-prepared reagents and ease of transportation (not shown in the drawings). The foil cover may be designed to be instantly removed or to sequentially open the sections (i.e. the foil cover is removed sequentially), or pierced. Before inserting the cartridge into the device, the foil above the section where the patient's swabs are to be placed is removed. The remaining foil is removed when the cartridge is installed in the device. The foil can be removed by pulling the tab on the side.

According to the embodiment shown in Fig. 10, the cartridge 36 is equipped with a transparent silicone film 94 with valves 96 juxtaposing each well opening 98 such that these valves form an air- and liquid-tight seal unless mechanical pressure from the dispenser 24 is applied, in which case the wells 50 of the cartridge 36 may receive liquid from the dispenser, however, the valve forms an air- and liquid-tight seal with a conical nozzle 100 forming the tip of the dispenser, as shown in the middle and on the right side of Fig. 10, illustrating the subsequent steps of the tip penetrating the film. This configuration creates a closed-system cartridge, which may be beneficial in handling highly dangerous pathogens, transporting samples or discarding used cartridges. Importantly, the film has substantial structural robustness to open specifically in valves and also substantial transparency to allow optical reactions readout.

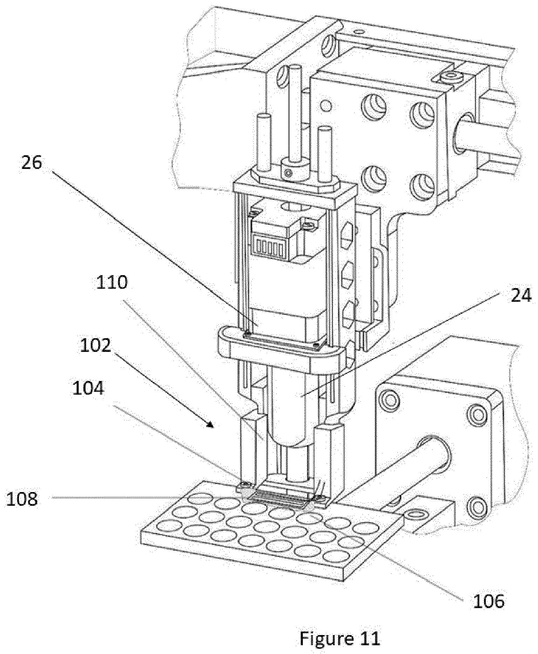

According to the embodiment shown in Fig. 11, the dispenser 24 is coupled with a sealing unit 102 of a film-sealing mechanism. This sealing unit comprises an aluminum grille 104, thermal insulation pads 106, and a heating element 108. The aluminum grille 104 is positioned by the dispenser positioning system 26 as well as the release bracket 110 (where it is immediately situated) to a desired location in the device and above a layer of film. The grille 104 is then pressed against the film by the dispenser positioning system 26 and a thermal protocol is executed, whereby the film melts and forms a hermetic seal with the underlying structure - for example, the cartridge.

The following features of the cartridge can be distinguished:
- wells differ in their shape (incl. size, volume and depth); their shape is determined by the volume of the liquid or material they are expected to hold and by additional functional requirements;
- the number of wells varies depending on the type of cartridge: as cartridges can be designed to accept samples from varying numbers of patients and the executed biochemical reactions can also vary, requiring different reagents (and correspondingly different volumes of reagents);
- wells are divided by functionality: reaction wells (serve as containers for carrying out the ultimate biochemical reaction of the analysis; these reactions may be temperature-dependent in which case the wells will be grouped to sit the thermal transfer plate once the cartridge is installed), accessory transfer wells (container for any type of additional plastic, metal or glass elements that are necessary for the analysis), reagent transfer wells (containers that are filled up with reagents prior to the installation of the cartridge into the device), sample receiving wells (containers, where an operator would place, dispense or rinse the sample), and waste wells (containers that will store liquids that are formed as waste in the process of sample preparation).

By using the cartridge described above, the device can perform:
- Nucleic acid extraction and purification in line with protocols certified for IVD, including parallel sample preparation;
- PCR, isothermal nucleic acid amplification, and ELISA;
- Colorimetric assays; and
- 3D-bioprinting

This implementation is based on a 49-reaction well thermal system, thus having the following quantitative properties:
- up to 49 separate reactions per cartridge, or 196 reactions per device (in a 4-cartridge batch loading);
- identifying up to 196 molecular targets (e.g. pathogens) per cartridge when the reactions are done in a multiplex manner when performing a PCR or isothermal amplification analysis;
- identifying up to 49 molecular targets per cartridge when performing ELISA

In order to illustrate the device's functionale, a specific usage example is given further. The device executes the following complete algorithm: an analysis of 4 patients for 7 pathogens each using the LAMP isothermal amplification reaction.
A. The operator collects nasal and throat swabs from 4 patients, peels off the cartridge foil cover and sequentially rinses the swabs in dedicated wells of the cartridge. The user interface of the device is used to register the samples and guide the operator to ensure sample collection and insertion. The operator closes the device door and launches the analysis from the user interface.
B. Inside the device, the dispenser and its dispenser positioning system execute a pre-defined algorithm, which includes the dispenser picking up a single-use plastic dispenser tip and executing a nucleic acid extraction and purification protocol as outlined above. At a technical level, this is a series of liquid transfers by the dispenser in the transfer, sample and waste wells. The dispenser works in close coordination with the magnetic assembly, which performs a series of proximity movements outlined above, and with the secondary heating module. Ultimately, the purified nucleic acid samples are transferred by the dispenser into reaction wells.
C. The primary thermal module launches an isothermal protocol and levels the temperature at 65°C for a duration of 20 minutes. This temperature is transferred to the contents of the reaction wells allowing the reaction to commence. In this period, the optical block acquires images of the reaction wells every 30 seconds in a light channel corresponding to the dye used. The incoming images are processed in the central computing unit and evaluated for signal intensity increase (an indication of ongoing amplification). The user interface reflects the reaction progression intensity in real-time.
D. Upon analysis completion, a final results table is shown on the monitor. The UV lamps light up to sterilize the compartment. Once done, the operator removes and disposes of the cartridge.

### List of reference numerals

- 10: housing
- 12: housing interior
- 14: display
- 16a, 16b: compartments
- 18a, 18b: sockets
- 20: front side
- 22: rear side
- 24: dispenser
- 26: dispenser positioning system
- 28: optical system
- 30: UV lamp
- 32: thermal control unit
- 30: UV lamp
- 32: thermal control unit
- 34: primary control unit module
- 36: cartridge
- 38a, 38b: screw-nut type linear drives
- 40a, 40b: threaded screw shaft
- 42a, 42b: motion drive
- 44: retention bracket of socket
- 45: work volume
- 46: piston of dispenser
- 48: motorized drive; stepper motor of dispenser
- 50: cartridge wells
- 52: energy transfer element
- 54: Peltier element
- 56: energy dissipation radiator
- 58: secondary thermal control unit module
- 60: heat exchange interface of secondary thermal control unit module
- 62: heating element of secondary thermal control unit module
- 64: heatsink
- 66: fan
- 68: magnetic unit
- 70a, 70b: magnetic elements
- 72: curved bracket
- 74: wall of well
- 76: linear drive
- 78: stepper motor of magnetic module
- 80: CCD camera
- 82: optical cubes
- 83: mirror
- 84: motor of the optical system
- 86: reaction wells
- 88: transfer wells
- 90: waste wells
- 92: sample receiving wells
- 93: accessory transfer wells
- 94: silicone film
- 96: valve
- 98: well opening
- 100: tip of dispenser
- 102: film-sealing mechanism
- 104: aluminum grille
- 106: thermal insulation pads
- 108: heating element of film-sealing mechanism
- 110: release bracket

## Claims

1. A point-of-care device for carrying out a variety of different biochemical reactions including the pre-treatment of an input material comprising a sample and/or a reactant to be subjected to a biochemical reaction, the device comprising
- a housing (10), which encloses a housing interior (12) accessible to an operator of the device and closeable in an airtight manner, the housing interior (12) comprising at least one compartment (16a, 16b) containing at least one socket (18a, 18b) adapted to receive a cartridge (36) comprising wells (50),
- a dispenser (24) arranged in the compartment (16a, 16b), said dispenser being designed to transfer a liquid between wells (50) of the cartridge (36) and optionally mix liquids for providing an input material in at least some of the wells, the dispenser (24) being arranged on a motion drive (42a, 42b) for its movement in at least two coordinate axes to displace the dispenser (24) in accordance with the coordinates of the location of each well (50) of the cartridge (36),
- at least one thermal control unit (32) comprised in the socket (18a, 18b) and comprising energy transfer elements (52) adapted to be brought into contact with at least one well (50) of the cartridge (36) via a thermal interface of the socket (18a, 18b), and
- an optical system (28) arranged in the compartment (16a, 16b) for the optical determination of the content of wells (50),
wherein the device further contains
- a magnetic unit (68) being arranged such to lie below the cartridge (36) in the state in which the cartridge is received in the socket (18a, 18b), said magnetic unit (68) comprising at least one magnetic element (70a, 70b) and being adapted to move the at least one magnetic element (70a, 70b) in relation to at least one of the wells (50) from a distal position to a proximal position, with the magnetic element being in closer proximity to the wall of the well (50) in the proximal position than in the distal position, and
- a UV lamp (30) for sterilizing the compartment (16a, 16b).

2. The device according to claim 1, wherein the thermal interface is formed by the inner wall of an indentation formed in the socket (18a, 18b) and adapted to firmly receive a respective well (50) of the cartridge (36), at least the lower portion of the well having a size and shape corresponding to the size and shape of the indentation, such that the inner wall of the indentation and the outer wall of the well are at least partially in direct contact with each other.

3. The device according to any of the preceding claims, the thermal control unit (32) comprising a primary control unit module (34) and a secondary control unit module (58), the primary control unit module being destined to run a temperature program for the pre-treatment of the input material by lysis of a biological material, and a secondary control unit module for carrying out the biochemical reaction, specifically a PCR of the prepared input material.

4. The device according to any of the preceding claims, wherein the dispenser (24) comprises at least one work volume (45) and a dispenser piston (46) located inside the work volume for metered extrusion of a liquid volume into a well (50) of the cartridge (36), the dispenser piston (46) being connected to a motorized drive (48) for its motion.

5. The device according to any of the preceding claims, further comprising a sealing unit (102) capable of placing a sealing film over designated parts of the cartridge (36) and applying pressure and thermal energy to the sealing film to create a hermetic seal encapsulating at least some of the wells (50).

6. The device according to any of the preceding claims, wherein the dispenser (24) comprises a conical nozzle (100) designed such to penetrate a plastic film covering at least one of the wells (50), whereby during penetration an airtight, reversible seal between the nozzle and the film is formed.

7. A cartridge for use in the device according to any of the preceding claims, the cartridge (36) containing a working part, which is essentially plane and in which invaginations are formed each defining a well (50) of the cartridge, the wells comprising an inlet opening (98) arranged in the plane of the working part, the shape of the inlet openings, the size of the inlet openings and/or the depth of at least some of the wells (50) differing from each other.

8. The cartridge according to claim 7, comprising wells of the following type:
A - a well designed for the execution of the biochemical reaction;
and wells of at least one of the following types:
B - a well designed for containing the necessary reagent to be transferred to the well according to A;
C - a well designed for containing consumable plastic elements;
D - a well designed for receiving samples to be analysed; and
E - a well designed for receiving waste;
wherein the size and shape of the wells according to A, B, C, D and E differ from each other.

9. The cartridge according to claim 7 or 8, wherein the top of the cartridge (36) is sealed for transportation in a filled and ready for use state, the seal being adapted such to allow the successive opening of different wells and/or different blocks of wells.

10. The cartridge according to claim 7 to 9, **characterized in that** the top of the cartridge (36) is sealed with a plastic film (94), preferably a silicone film, with valves (96) being arranged in the film, said valves containing valve openings designed such that the valves are air- and liquid-tight unless the dispenser (24) applies pressure, in which case the valve (96) forms an airtight seal with a tip of the dispenser but allows liquid to be transferred from the dispenser into the respective well.

11. The cartridge according to claim 7 to 10, wherein the cartridge body is equipped with an electronic identification tag, which contains information for allowing to identify at least the cartridge model.

12. A point-of-care system for carrying out a biochemical reaction including the pre-treatment of an input material comprising a sample and/or a reactant to be subjected to a biochemical reaction, the system comprising the device according to any of claims 1 to 6 and the cartridge according to any of claims 7 to 11.

13. A process of carrying out a biochemical reaction using the device according to any of claims 1 to 6 and the cartridge according to any of claims 7 to 11, the process comprising the step of
a. introducing the cartridge containing the samples and/or reagents into the compartment and placing it in the at least one socket;
b. transferring liquid between wells of the cartridge and optionally mix liquids such to provide a reaction input material containing a predetermined amount of a sample and/or reagent in at least some of the wells;
c. setting at least some of the wells to a specific temperature using the at least one thermal control unit
d. optionally subjecting the reaction input material to a magnetic pre-treatment using the magnetic unit;
e. determining the content of wells using the optical system, and
f. sterilizing the compartment using the UV lamp.

14. The process according to claim 13, the biochemical reaction being at least one selected from the group consisting of qualitative and quantitative real-time nucleic acid amplification detection, colorimetric assays and its sample preparation, ELISA assays and its sample preparation, culture assays and 3D bioprinting.
